# EUROPÄISCHE PATENTSCHRIFT

(11) **EP 0 699 455 B1**
(45) Veröffentlichungstag und Bekanntmachung des Hinweises auf die Patenterteilung: **03.05.2000**
(21) Anmeldenummer: 95112170.6
(22) Anmeldetag: 02.08.1995
(51) Int. Cl.: A61N 5/06, B08B 3/02

(54) **Ganzkörper-Bräunungsgerät**
Device for tanning the whole body
Appareil de bronzage du corps entier

(30) Priorität: 30.08.1994 DE 4430815
(43) Veröffentlichungstag der Anmeldung: 06.03.1996
(73) Patentinhaber: JK-JOSEF KRATZ GmbH, 53578 Windhagen (DE)
(72) Erfinder: Kratz, Walter, D-53783 Eitorf (DE)
(74) Vertreter: Heim, Hans-Karl, Dipl.-Ing.

(56) Entgegenhaltungen:
- EP-A- 0 334 979
- US-A- 5 240 503

## Beschreibung

Die Erfindung betrifft ein Ganzkörper-Bräunungsgerät mit einer Liegefläche oder mit einer Liegefläche mit Kopfpolster.

Ein Problem im öffentlichen oder kommerziellen Betrieb eines Ganzkörper-Bräunungsgerätes ist eine ausreichende Hygiene der Liegefläche. Die Liegefläche, die in der Regel aus Kunstglas besteht, muß nach jeder Benutzung mit einem Reinigungs- oder Desinfektionsmittel gereinigt werden. Dies geschieht bisher manuell entweder durch den Betreiber oder den Nutzer des Ganzkörper-Bräunungsgerätes. Es liegt auf der Hand, daß bei einer derartigen manuellen Tätigkeit das Reinigungsergebnis nicht gleichmäßig und in vielen Fällen auch nicht optimal ist. Durch diese ungleichmäßige und vor allem nicht kontrollierbare Reinigung ist das Vertrauen des Nutzers in die Sauberkeit der Liegefläche eingeschränkt. Gerade in kommerziell betriebenen Geräten ist jedoch das Vertrauen und die Zufriedenheit der Nutzer mit den angebotenen Bedingungen von wesentlicher Bedeutung.

Aus der EP 0 334 979 A1 ist ein Bräunungsgerät mit einer Auflageplatte bekannt, welche auf einem oder mehreren Kipprahmen aufliegt. Die Kipprahmen sind verschwenkbar angeordnet, um die Liegestellung der zu bestrahlenden Personen deren individuellen Wünschen anpassen zu können. Oberhalb und beabstandet zu einer Auflageebene der Auflageplatte ist mattenartig ein Aufliegetuch gespannt, welches als Endlosband ausgebildet ist und mit Hilfe von Führungseinrichtungen und Umlenkrollen um die Auflageplatte geführt wird. Unterhalb der Auflageplatten und des Rahmengestells sind eine Reinigungseinrichtung und eine Trockeneinrichtung derart angeordnet, daß das Aufliegetuch nacheinander in beide Einrichtungen hinein- und herausbewegt werden kann.

Das bekannte Bräunungsgerät mit dem hängemattenähnlich gespannten Aufliegetuch weist eine aufwendige Konstruktion und einen relativ zeitaufwendigen Reinigungszyklus auf. Die zusätzlichen Einrichtungen für das endlose Aufliegetuch und die Reinigungs- und Trockeneinrichtung erfordern außerdem einen relativ großen Platzbedarf.

Eine aus der US-PS 5,240,503 bekannte, aufwendig konstruierte automatische Vorrichtung ist für Fassadenerneuerungen, z.B. durch Sandstrahlen und Anstreichen, von Wolkenkratzern und Hochhäusern vorgesehen und kann auch zur Reinigung der Gebäudefenster eingesetzt werden.

Der Erfindung liegt die **Aufgabe** zugrunde, die Liegefläche eines Ganzkörper-Bräunungsgerätes nach jeder Benutzung schnell, zuverlässig und kontrollierbar zu reinigen.

Die Lösung dieser Aufgabe erfolgt gemäß den Merkmalen des Anspruchs 1. Vorteilhafte Weiterbildungen sind in den abhängigen Ansprüchen beschrieben.

Erfindungsgemäß weist ein Bräunungsgerät mit einer Liegefläche und einer automatischen Reinigungsvorrichtung für die Liegefläche wenigstens eine Auslaßöffnung zum Aufbringen eines Reinigungsmittels auf die Liegefläche und einer Einrichtung zum Entfernen des Reinigungsmittels von der Liegefläche auf. Nach einem weiteren Grundgedanken der Erfindung kann die Zeitdauer zwischen Aufbringen und Entfernen des Reinigungsmittels eingestellt werden.

Es ist vorteilhaft, wenn die Reinigungsvorrichtung einen fahrbaren Querbalken aufweist, welcher über die Liegefläche fährt und dabei ein Reinigungsmittel auf diese aufbringt. Das Reinigungsmittel kann eine definierte Zeit auf die Liegefläche einwirken, um so eine Reinigung und Desinfektion zu bewirken. In einem zweiten Arbeitsgang fährt der Querbalken zurück und entfernt dabei das Reinigungsmittel wieder von der Liegefläche.

Dadurch erfolgt eine automatisierte Reinigung des Gerätes in hoher Qualität und in für den Nutzer nachvollziehbarer Gleichmäßigkeit, wodurch sich sein Sicherheitsgefühl und sein Wohlbefinden bei der Nutzung des Gerätes erhöht. Zudem reduziert die Erfindung den Arbeitsaufwand des Betreibers und den Papierverbrauch, der durch die manuelle Reinigung entsteht, und führt so zu einer höheren Wirtschaftlichkeit. Ein weiterer Vorteil ist die Reduzierung der Stillstandszeit, die zu einer Erhöhung der Nutzungsfrequenz führt.

Ein besonderes Problem bei der Reinigung der Liegefläche stellt die Reinigung eines Kopfpolsters dar. Prinzipiell ist es natürlich möglich, das Kopfpolster als getrenntes Teil zur Verfügung zu stellen und einfach auszuwechseln oder getrennt zu reinigen, wodurch jedoch eine Reihe der Vorteile der automatischen Reinigung verlorengehen. Günstig ist die Anordnung des Kopfpolsters unter einem dünnen, flexiblen und elastisch dehnbaren Material, welches im folgenden als Membran bezeichnet wird.

Die Liegefläche kann auch einen weichen Flächenbereich in der Ebene der Liegefläche aufweisen, wobei auf ein erhöhtes Kopfpolster verzichtet wird. So ist eine problemlose Reinigung der gesamten Liegefläche einschließlich der weichen Kopfauflage möglich.

In einer vorteilhaften Weiterbildung ist unter dem Kopfpolster ein Stempel angeordnet, der im ausgefahrenen Zustand die Membran des Kopfpolsters in eine gegenüber der Liegefläche erhöhte Position bringt. Dadurch bildet das von der Membran überspannte Kopfpolster während der Nutzung die typische Kopfpolsterform, die einen hohen Liegekomfort gewährleistet. Für den Reinigungsvorgang fährt der Stempel zurück, und die Oberfläche des Kopfpolsters, also die Membran, liegt dann in einer Ebene mit der Liegefläche.

In einer anderen Ausführungsform ist unter der Membran des Kopfpolsters eine Überdruckeinrichtung vorhanden, durch welche die Membran nach oben zu wölben ist. Dadurch wird die Membran gegen die Reinigungsvorrichtung gedrückt, ohne ein nicht überwindbares Hindernis zu sein. Ein weiterer Vorteil dieser Ausführungsform ist, daß keine aufwendige Mechanik unter der Liegefläche zur Bewegung des Kopfpolsters notwendig ist.

In einer vorteilhaften Ausgestaltung der Erfindung weist die Reinigungsvorrichtung einen über die Liegefläche fahrbaren Balken auf. In dieser geometrisch einfachen Form, können die verschiedenen funktionalen Einrichtungen gut angeordnet werden.

In einer vorteilhaften Weiterbildung weist die Reinigungsvorrichtung mindenstens eine Düse als Auslaßöffnung zum Aufsprühen eines Reinigungmittels auf. Da die verwendeten Reinigungs- und Desinfektionmittel in der Regel flüssig sind, ist die Verwendung von einer oder mehrerer Düsen zum Aufbringen des Reinigungsmittels auf die Liegefläche besonders vorteilhaft.

In einer anderen Weiterentwicklung weist die Erfindung eine Gummilippe zum Abschieben oder Abziehen des Reinigungsmittels auf. Dadurch wird im zweiten Arbeitsgang, also beim Zurückfahren der Reinigungsvorrichtung, auf einfache Weise ein Entfernen des Reinigungsmittels und damit ein Trocknen der Liegefläche bewirkt. In einer vorteilhaften Weiterbildung ist die Gummilippe V-förmig oder bogenförmig ausgebildet, so daß das Reinigungsmittel sich an einem definierten Punkt oder zumindest in einem vorgegebenen Bereich der Gummilippe sammelt. Dadurch wird vermieden, daß das Reinigungsmittel an den Seiten wegfließt, und außerdem wird es an einem Punkt gesammelt, an dem es dann leicht abgeführt werden kann. So kann die Reinigungsvorrichtung eine Unterdruckeinrichtung zum Absaugen des Reinigungsmittels aufweisen, welche das Reinigungsmittel auf der gesamten Liegefläche oder im Zusammenwirken mit der Gummilippe an einem bestimmten Punkt absaugt.

In einer vorteilhaften Weiterbildung kann die Reinigungsvorrichtung ein Gebläse zum Trocknen der Liegefläche aufweisen. Dieses Gebläse kann alternativ oder im Zusammenwirken mit der Gummilippe oder der Unterdruckeinrichtung eingesetzt werden, um so zu einer schnelleren Trocknung der Liegefläche zu führen. Das Gebläse kann zusätzlich eine Heizeinrichtung aufweisen, um die Luft zu erwärmen.

In einer anderen Weiterbildung ist die Liegefläche mit einem Behälter zur Aufnahme des abgeschobenen Reinigungsmittels ausgestattet. Dieser ist günstigerweise am Ende der Liegefläche angeordnet und nimmt das Reinigungsmittel auf oder führt es mittels eines Leitungssystems zu einem unter der Liegefläche angeordneten Sammelbehälter weiter.

In einer anderen Ausführungsform sind an den Seiten oder wenigstens an einer Seite der Liegefläche Rinnen oder Kanäle angeordnet, die das Reinigungsmittel aufnehmen. Falls beim Abschieben des Reinigungsmittels Flüssigkeit an den Seiten abläuft, spritzt es nicht auf den Boden, sondern wird in den Kanälen gesammelt und zu einem Behälter geführt.

In einer Weiterbildung der Erfindung ist eine mit der Reinigungsvorrichtung verbundene optische Anzeige vorgesehen, so daß der Reinigungsvorgang visuell anzeigbar ist. Dies kann z.B. durch eine Funktionslampe oder eine elektronische Anzeige geschehen, wodurch einer Störung des Reinigungsvorganges vorgebeugt wird. Durch eine folgende Anzeige des gereinigten Status wird das Vertrauen des Nutzers erhöht.

In einer anderen Weiterbildung ist mit der Reinigungsvorrichtung ein Reinigungsmitteln mit Duftstoffen auf die Liegefläche aufbringbar. Durch eine solche positive Stimulation der Geruchsnerven nimmt der Nutzer die vorausgegangene Reinigung im Unterbewußtsein zur Kenntnis und sein Wohlbefinden wird erhöht.

In dem die Zeitdauer zwischen Aufbringen und Entfernen des Reinigungsmittels einstellbar ist, wird sichergestellt, daß das Reinigungs- oder Desinfektionsmittel bis zur vollständigen Desinfektion der Liegefläche auf diese einwirken kann.

In einer vorteilhaften Weiterentwicklung ist eine Datenverbindung zwischen der Reinigungsvorrichtung und dem Ganzkörper-Bräunungsgerät vorgesehen, so daß die Reinigungsvorrichtung durch Betriebsende des Ganzkörper-Bräunungsgeräts aktivierbar ist. Durch eine solche Steuerung entfällt die manuelle Bedienung und die Stillstandszeit des Gerätes verkürzt sich. So kann das Gerät besser ausgelastet und genutzt werden. Die Elektronik der Steuerung der Reinigungsvorrichtung kann auch eine Schnittstelle für den Anschluß an eine PC-Studiosteuerung aufweisen. So können an einer zentralen Stelle verschiedene Funktionen und Statusanzeigen der Reinigungsvorrichtung abgerufen werden. So kann dort der Reinigungsvorgang selbst oder auch das erwartete Ende des Reinigungsvorgangs und damit ein Freiwerden und eine erneute Nutzung des Ganzkörper-Bräunungsgerätes angezeigt werden. Auch die Anzeige möglicher Wartungsarbeiten, wie das Nachfüllen von Reinigungsmittel oder Wasser, ist sinnvoll.

In einer vorteilhaften Ausführungsform ist die Reinigungsvorrichtung senkrecht zur Längsachse der Liegefläche ausgerichtet. Eine Reinigungsvorrichtung, die parallel zur Längsachse der Liegefläche ausgerichtet ist, ist allerdings ebenso denkbar.

In einer anderen Weiterbildung weist die Reinigungsvorrichtung eine Reinigungsleiste oder -walze auf, die zumindest zeitweise im berührenden Kontakt mit der Liegefläche ist. Diese Reinigungsleiste kann als Walzenbürste, Schaumstoffwalze oder als ein mit Schaumstoff umspannter Balken ausgebildet sein. Um die Wirkung dieser Walze zu steigern, ist eine Rotation oder eine seitliche Hin- und Herbewegung dieser Walze vorteilhaft. Durch diese mechanische Reinigung wird das Reinigungsmittel besser verteilt, wirkt besser auf die Liegefläche ein und ist insbesondere dann hilfreich, wenn sich der Nutzer eingecremt hat und Fettrückstände von der Liegefläche zu beseitigen sind.

Die Reinigungsvorrichtung kann in der Weise weitergebildet werden, daß die Reinigungswalze spezielle Borsten aufweist, mit denen eine Politur der Liegefläche möglich ist. Da die Liegefläche in der Regel aus Acrylglas besteht, ist es vorteilhaft, diese immer wieder zu polieren, weil Acrylglas sich schnell abnutzt. Bei ständiger Beanspruchung entstehen auf der Liegefläche matte Stellen, die zum einen weniger UV-Licht durchlassen und zum anderen dem Benutzer das Gefühl vermitteln, ein nicht bestmöglich gepflegtes Bräunungsgerät zu benutzen. Deshalb ist es erfindungsgemäß vorgesehen, die Reinigungsvorrichtung so auszubilden, daß die Liegefläche ungefähr einmal täglich, und zwar in der Regel abends nach Betriebsende, poliert wird. Dazu wird eine Reinigungswalze mit speziellen Borsten ausgestattet, die mit einer Polierpaste die Liegefläche polieren. Die Polierpaste kann entweder aus der Reinigungswalze heraus in die Borsten gespritzt werden oder aus der Auslaßöffnung der Reinigungsvorrichtung auf die Liegefläche aufgebracht werden.

In einer anderen Ausbildungsform ist die Auslaßöffnung der Reinigungsvorrichtung oder eine zusätzlich vorgesehene Düse als Dampfdüse ausgebildet. Erfindungsgemäß kann ein Dampfgerät in der Reinigungsvorrichtung vorhanden sein, welches als Reinigungsmittel Dampf auf die Liegefläche aufbringt. Durch Dampf wird die Liegefläche von Fett gereingt und gleichzeitig desinfiziert. Dadurch kann der Einsatz teurer chemischer Reinigungsmittel reduziert oder vermieden werden.

Vorteilhaft ist die Kombination dieser Dampfreinigung mit einer Reinigungsleiste, die ein textiles Material aufweist, um die Liegefläche nach einer Dampfbehandlung mechanisch nachzubehandeln. Vorgesehen ist die Verwendung eines weichen Materials wie Frotteestreifen.

Nachfolgend wird die Erfindung anhand einer Zeichnung weiter erläutert. Im einzelnen zeigen die Darstellungen in:
- Fig. 1: einen schematisch dargestellten Gesamtaufbau einer Liegefläche mit einer erfindungsgemäßen Reinigungsvorrichtung;
- Fig. 2: einen Querschnitt durch eine erfindungsgemäße Reinigungsvorrichtung;
- Fig. 3: einen vertikalen Querschnitt durch eine Liegefläche mit Kopfpolster gemäß der Erfindung und
- Fig. 4: einen vertikalen Querschnitt durch eine andere Ausführungsform des Kopfpolsters in der Liegefläche.

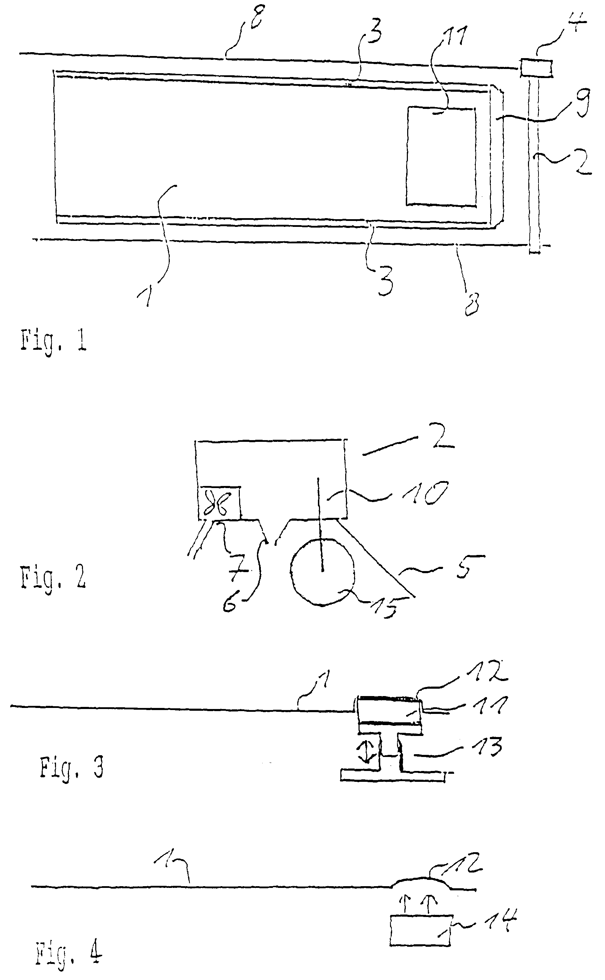

Fig. 1 zeigt einen schematisierten Gesamtaufbau einer Liegefläche 1 eines Ganzkörper-Bräunungsgeräts mit Kopfpolster 11 und einer Reinigungsvorrichtung 2. Die Reinigungsvorrichtung 2 bewegt sich parallel zur Längsachse der Liegefläche 1 über die Liegefläche, um in einem ersten Arbeitsgang ein Reinigungsmittel auf die Liegefläche aufzubringen und in einem zweiten Arbeitsgang, sozusagen beim Zurückfahren, dieses Reinigungsmittel wieder zu entfernen. Die Zeitdauer zwischen Aufbringen und Entfernen ist einstellbar, um eine gründliche Desinfektion der Liegefläche 1 zu gewährleisten. Die Reinigungsvorrichtung 2 weist einen Motor 4 auf, der die Reinigungsvorrichtung 2 entlang der Gleitschienen 8 über die Liegefläche 1 und das Kopfpolster 11 bewegt. Die Reinigungsvorrichtung 2 kann sowohl gleitend oder rollend auf den Schienen 8, als auch aufgehängt unter den Schienen 8 angeordnet sein. Der Motor 4 wird von einer elektronischen Steuerung geregelt, die auch die Zeitdauer zwischen Aufbringen und Entfernen des Reinigungsmittels überwacht und mit dem Ganzkörper-Bräunungsgerät verbunden ist, um eine automatische Reinigung nach dem Betrieb des Ganzkörper-Bräunungsgerätes auszulösen. An den Seiten der Liegefläche 1 sind Rinnen 3 ausgebildet, die beim Entfernen des Reinigungsmittels an den Seiten der Reinigungsvorrichtung 2 ablaufende Flüssigkeit aufnehmen und in einen Behälter 9 abführen. Der Behälter 9 ist am Ende der Liegefläche 1 angeordnet und dient auch zur Aufnahme der Flüssigkeit, die die Reinigungsvorrichtung 2 beim Zurückfahren auf der Liegefläche 1 vor sich herschiebt. An Stelle des Behälters 9 kann dort auch eine Sammelvorrichtung mit einer Zuleitung zu einem unter der Liegefläche angeordneten Behälter vorhanden sein.

Fig. 2 zeigt einen Querschnitt durch eine Reinigungsvorrichtung 2, die einen fahrbaren Balken 10 aufweist, der dann über die in Fig. 1 gezeigte Liegefläche 1 bewegt wird. An der Unterseite des fahrbaren Balkens 10 ist eine als Düse dargestellte Auslaßöffnung 6 angeordnet. Die Zuleitung des Reinigungsmittels erfolgt über die Reinigungsvorrichtung 2, in der entweder ein Behälter vorhanden ist oder eine Zuleitung zu einem außerhalb angeordneten Behälter. Die Reinigungsvorrichtung 2 weist eine Einrichtung 5 zum Entfernen der Flüssigkeit von der Liegefläche auf, die in dieser Ausführung als Gummilippe ausgebildet ist. Diese ist nicht senkrecht, sondern in einem Winkel zur Oberfläche der Liegefläche 1 angeordnet, um auf diese Weise einen besseren Kontakt zur Liegefläche 1 zu haben. Günstigerweise ist die Gummilippe 5 gebogen oder gekrümmt ausgebildet, um das Reinigungsmittel in einem vorgegebenen Bereich zu sammeln. Dadurch wird ein übermäßiges Ablaufen an den Seiten verhindert und eine Aufnahme in einen Behälter erleichtert. Man kann sich auch vorstellen, daß die Flüssigkeit, die vor der Gummilippe gesammelt wird, nach oben in die Reinigungsvorrichtung 2 abgesaugt wird. Aus der Auslaßöffnung 6 tritt Reinigungsmittel aus, welches auf die Oberfläche der Liegefläche 1 gelangt. In Längsrichtung der Reinigungsvorrichtung 2 kann eine oder eine Vielzahl von Düsen angeordnet sein, die auch in verschiedenen Winkeln und unter Druck das Reinigungsmittel auf die Liegefläche aufbringen. Außerdem ist in der Reinigungsvorrichtung 2 ein Gebläse 7 mit einem düsenförmigen Austritt vorhanden, welches Luft oder auch erwärmte Luft auf die Liegefläche 1 des Ganzkörperbräunungsgerätes strömen läßt. Dadurch kann eine zusätzliche Trocknung der abgeschobenen Liegefläche erfolgen. Wenn die Liegefläche unter ergonomischen Gesichtspunkten mit Ausbuchtungen und Vertiefungen gestaltet ist, ist dieses Vorgehen vorteilhaft. Wenn das Gebläse 7 stark genug ausgebildet ist, kann die Trocknung auch einzig und allein durch das Gebläse 7 erfolgen, welches eventuell mit einer zusätzlichen Heizeinrichtung versehen ist. Bei einer ergonomischen Liegefläche ist aber auch die Benutzung einer segmentierten Gummilippe als Abschiebeeinrichtung möglich. Eine solche in direkt nebeneinander liegende Abschnitte unterteilte Gummilippe, die sich dadurch den einzelnen Mulden in einer ergonomischen Liegefläche anpassen kann, ist besonders wirkungsvoll im Zusammenspiel mit einer zweiten, nachgeordneten oder vorausgehenden Gummilippe, die nicht segmentiert oder deren Segmentierung versetzt zu den Segmenten der ersten Gummilippe ausgebildet ist. Die Walze 15 ist mit der Reinigungsvorrichtung 2 verbunden und kann abgesenkt oder heraufgefahren werden. Beim Aufsprühen des Reinigungsmittels wird die Walze 15 herabgesenkt und bearbeitet die Liegefläche 1. Dies kann durch Rotation oder durch eine seitliche Hin- und Herbewegung geschehen. Die Oberfläche der Walze 15 ist aus einem flexiblen, weichen, zur Reinigung geeignetem Material ausgebildet. Dies kann entweder eine Bürstenwalze sein oder eine mit Schaumstoff oder einem anderen Material überspannte Walze oder Leiste.

In Fig. 3 wird ein Querschnitt durch eine Liegefläche 1 mit Kopfpolster 11 dargestellt. Das Kopfpolster 11 ist im Bereich eines Endes der Liegefläche 1 angeordnet. Zur Integration in die Liegefläche 1 ist eine Membran 12, die mit der Liegefläche 1 verbunden ist, oberhalb des Kopfpolsters 11 angeordnet. Das weiche Material des Kopfpolsters 11 wird also von der Membran 12 überzogen und von ihr geschützt. Um einerseits das Kopfpolster 11 zur Erhöhung des Liegekomforts in eine erhöhte Lage zu bringen, und andererseits bei dem Reinigungsvorgang das Kopfpolster 11 fluchtend in die Ebene der Liegefläche 1 zu integrieren, ist unterhalb des Kopfpolsters 11 ein Stempel 13 angeordnet. Dieser Stempel 13 ist mit der Reinigungsvorrichtung 2 verbunden und wird automatisch vor Beginn des Reinigungsvorgangs angesteuert, um das Kopfpolster 11 abzusenken.

Fig. 4 zeigt eine andere Ausführungsform einer erfindungsgemäßen Liegefläche 1 mit Kopfpolster. In dieser Ausführungsform ist eine straff gespannte Membran 12 in der Ebene der Liegefläche 1 vorhanden. Während der Benutzung des Ganzkörper-Bräunungsgerätes erzeugt eine Überdruckeinrichtung 14, die unterhalb der Membran 12 angeordnet ist, eine Luftströmung nach oben, welche die Membran nach oben wölbt und so für den Nutzer eine erhöhte Lage des Kopfpolsters erreicht. Während des Reinigungsvorgangs erzeugt die Überdruckeinrichtung 14 keinen Überdruck und die Membran 12 liegt in der Ebene der Liegefläche 1 und kann leicht mit der Reinigungsvorrichtung 2 gereinigt werden.

## Patentansprüche

1. Ganzkörper-Bräunungsgerät mit einer Liegefläche (1) und einer automatischen Reinigungsvorrichtung (2) für die Liegefläche (1),
dadurch **gekennzeichnet**,
daß die Reinigungsvorrichtung (2) wenigstens eine Auslaßöffnung (6) zum Aufbringen eines Reinigungsmittels auf die Liegefläche (1) und eine Einrichtung (5) zum Entfernen des Reinigungsmittels von der Liegefläche (1) aufweist,
daß die Zeitdauer zwischen Aufbringen und Entfernen des Reinigungsmittels einstellbar ist.

2. Ganzkörper-Bräunungsgerät nach Anspruch 1,
dadurch gekennzeichnet,
daß die Reinigungsvorrichtung (2) parallel zur Längsachse der Liegefläche (1) über die Liegefläche (1) bewegbar ist.

3. Ganzkörper-Bräunungsgerät nach einem der vorhergehenden Ansprüche,
dadurch **gekennzeichnet,**
daß die Reinigungsvorrichtung (2) einen über die Liegefläche (1) fahrbaren Balken (10) aufweist.

4. Ganzkörper-Bräunungsgerät nach einem der vorhergehenden Ansprüche,
dadurch **gekennzeichnet,**
daß die Reinigungsvorrichtung (2) mindestens eine Düse (6) zum Aufsprühen eines Reinigungsmittels und/oder eines Duftstoffes auf die Liegefläche (1) aufweist.

5. Ganzkörper-Bräunungsgerät nach einem der vorhergehenden Ansprüche,
dadurch **gekennzeichnet,**
daß die Reinigungsvorrichtung (2) eine Gummilippe (5) zum Abschieben oder Abziehen des Reinigungsmittels aufweist, und
daß die Gummilippe (5) V-förmig oder bogenförmig ausgebildet ist, so daß sich das Reinigungsmittel beim Abschieben oder Abziehen an einem Punkt der Gummilippe (5) sammelt.

6. Ganzkörper-Bräunungsgerät nach einem der vorhergehenden H Ansprüche,
dadurch **gekennzeichnet,**
daß die Reinigungsvorrichtung (2) eine Unterdruckeinrichtung zum Absaugen des Reinigungsmittels und/oder ein Gebläse (7) zum Trocknen der Liegefläche (1) aufweist.

7. Ganzkörper-Bräunungsgerät nach einem der vorhergehen Ansprüche,
dadurch **gekennzeichnet,**
daß die Liegefläche (1) an wenigstens einer Seite eine Rinne oder einen Kanal (3) zur Aufnahme des Reinigungs-, mittels aufweist, das beim Abschieben oer Abziehen an den Seiten abfließt, und
daß die Liegefläche (1) mit einem Behälter (9) zur Aufnahme des abgeschobenen oder abgezogenen Reinigungsmittels ausgestattet ist.

8. Ganzkörper-Bräunungsgerät nach einem der vorhergehenden Ansprüche,
dadurch **gekennzeichnet**,
daß eine mit der Reinigungsvorrichtung (2) verbundene optische Anzeige vorgesehen ist, welche den Reinigungsvorgang visuell anzeigt.

9. Ganzkörper-Bräunungsgerät nach einem der vorhergehenden Ansprüche,
dadurch **gekennzeichnet,**
daß eine Verbindung zwischen der Reinigungsvorrichtung (2) und dem Ganzkörper-Bräunungsgerät vorgesehen ist, so daß die Reinigungsvorrichtung (2) durch Betriebsende des Ganzkörper-Bräunungsgerätes aktivierbar ist.

10. Ganzköper-Bräunungsgerät nach einem der vorhergehenden Ansprüche,
dadurch **gekennzeichnet,**
daß die Reinigungsvorrichtung (2) senkrecht zur Längsachse der Liegefläche (1) ausgerichtet ist.

11. Ganzkörper-Bräunungsgerät nach einem der vorhergehenden Ansprüche,
dadurch **gekennzeichnet,**
daß die Auslaßöffnung (6) oder eine zusätzlich vorgesehene Düse als Dampfdüse ausgebildet ist.

12. Ganzkörper-Bräunungsgerät nach einem der vorhergehenden Ansprüche,
dadurch **gekennzeichnet,**
daß die Reinigungsvorrichtung (2) mindestens eine Reinigungsleiste und/oder -walze (15) aufweist, welche zumindest zeitweise im berührenden Kontakt mit der Liegefläche (1) ist und welche Borsten und/oder ein textiles Material aufweist, so daß eine Politur und/oder eine mechanische Nachbehandlung der Liegefläche (1) nach einer Dampfbehandlung möglich ist.

13. Ganzkörper-Bräunungsgerät nach einem der vorhergehenden Ansprüche,
dadurch **gekennzeichnet,**
daß unter einer flexiblen Membran (12) ein Kopfpolster (11) angeordnet ist, und
daß das Kopfpolster (11) während eines Reinigungsvorganges in die Liegefläche (1) fluchtend integriert ist.

14. Ganzkörper-Bräunungsgerät nach Anspruch 2,
dadurch **gekennzeichnet,**
daß das Kopfpolster (11) als eine weiche Fläche in der Ebene der Liegefläche (1) angeordnet ist.

15. Ganzkörper-Bräunungsgerät nach Anspruch 2 oder 3,
dadurch **gekennzeichnet,**
daß unter dem Kopfpolster (11) ein Stempel (13) und/oder eine Überdruckeinrichtung (14) angeordnet ist, durch welche die Membran (12) des Kopfpolsters (11) in eine erhöhte Form bringbar ist.

## Claims

1. Device for tanning the whole body with a lying surface (1) and an automatic cleaning apparatus (2) for the lying surface (1),
**characterized** in that
the cleaning apparatus (2) has at least one outlet port (6) for the application of a detergent to the lying surface (1) and a device (5) for removing the detergent from the lying surface (1) and that the time period between the application and removal of the detergent is adjustable.

2. Device for tanning the whole body according to claim 1,
**characterized** in that
the cleaning apparatus (2) is movable over and parallel to the longitudinal axis of the lying surface (1).

3. Device for tanning the whole body according to one of the preceding claims,
**characterized** in that
the cleaning apparatus (2) has a beam (10) movable over the lying surface (1).

4. Device for tanning the whole body according to one of the preceding claims,
**characterized** in that
the cleaning apparatus (2) has at least one nozzle (6) for spraying a detergent and/or a perfume onto the lying surface (1).

5. Device for tanning the whole body according to one of the preceding claims,
**characterized** in that
the cleaning apparatus (2) has a rubber lip (5) for sliding or drawing off the detergent and that the rubber lip (5) has a V-shaped or arcuate construction so that on sliding or drawing off the detergent collects at one point of the rubber lip (5).

6. Device for tanning the whole body according to one of the preceding claims,
**characterized** in that
the cleaning apparatus (2) has a vacuum device for sucking off the detergent and/or a fan (7) for drying the lying surface (1).

7. Device for tanning the whole body according to one of the preceding claims,
**characterized** in that
on at least one side the lying surface (1) has a groove or channel (3) for receiving the detergent which flows out at the sides on sliding or drawing off and that the lying surface (1) is equipped with a container (9) for receiving the slid or drawn off detergent.

8. Device for tanning the whole body according to one of the preceding claims,
**characterized** in that
an optical display connected to the cleaning apparatus (2) is provided and visually displays the cleaning process.

9. Device for tanning the whole body according to one of the preceding claims,
**characterized** in that
there is a connection between the cleaning apparatus (2) and the whole body tanning device so that the cleaning apparatus (2) can be activated by the end of operation of the whole body tanning device.

10. Device for tanning the whole body according to one of the preceding claims,
**characterized** in that
the cleaning apparatus (2) is adjusted perpendicular to the longitudinal axis of the lying surface (1).

11. Device for tanning the whole body according to one of the preceding claims,
**characterized** in that
the outlet port (6) or an additionally provided nozzle is constructed as a steam nozzle.

12. Device for tanning the whole body according to one of the preceding claims,
**characterized** in that
the cleaning apparatus (2) has at least one cleaning strip and/or roller (15) which is at least temporarily in contact with the lying surface (1) and which has bristles and/or a textile material so that a polishing and/or a mechanical after-treatment of the lying surface (1) is possible following a steam treatment.

13. Device for tanning the whole body according to one of the preceding claims,
**characterized** in that
below a flexible membrane (12) is provided a pillow (11) and that the pillow (11) is integrated in aligned manner into the lying surface (1) during a cleaning process.

14. Device for tanning the whole body according to claim 13,
**characterized** in that
the pillow (11) is placed as a soft surface in the plane of the lying surface (1).

15. Device for tanning the whole body according to claim 13 or 14,
**characterized** in that
below the pillow (11) is located a plunger (13) and/or an overpressure mechanism (14) through which the membrane (12) of the pillow (11) can be brought into a raised form.

## Revendications

1. Appareil de bronzage intégral avec une couchette (1) et un dispositif de nettoyage automatique (2) pour la couchette (1),
**caractérisé** en ce que le dispositif de nettoyage (2) comporte au moins une ouverture de sortie (6) pour appliquer un agent de nettoyage sur la couchette (1) et un dispositif (5) pour éliminer l'agent de nettoyage de la couchette (1), et en ce que la durée écoulée entre l'application et l'élimination de l'agent de nettoyage est réglable.

2. Appareil de bronzage intégral selon la Revendication 1,
**caractérisé** en ce que le dispositif de nettoyage (2) est mobile au-dessus de la couchette (1) parallèlement à l'axe longitudinal de la couchette (1).

3. Appareil de bronzage intégral selon l'une quelconque des Revendications précédentes,
**caractérisé** en ce que le dispositif de nettoyage (2) comporte une poutrelle (10) mobile au-dessus de la couchette (1).

4. Appareil de bronzage intégral selon l'une quelconque des Revendications précédentes,
**caractérisé** en ce que le dispositif de nettoyage (2) comporte au moins une buse (6) pour pulvériser un agent de nettoyage et/ou un agent désodorisant sur la couchette (1).

5. Appareil de bronzage intégral selon l'une quelconque des Revendications précédentes,
**caractérisé** en ce que le dispositif de nettoyage (2) comporte une lèvre (5) en caoutchouc pour repousser ou racler l'agent de nettoyage, et en ce que la lèvre (5) en caoutchouc est conformée en V ou en arc, afin que l'agent de nettoyage se collecte en un point de la lèvre (5) en caoutchouc lors du repoussement ou du raclage.

6. Appareil de bronzage intégral selon l'une quelconque des Revendications précédentes,
**caractérisé** en ce que le dispositif de nettoyage (2) comporte un dispositif à dépression pour aspirer l'agent de nettoyage et/ou un ventilateur (7) pour sécher la couchette (1).

7. Appareil de bronzage intégral selon l'une quelconque des Revendications précédentes,
**caractérisé** en ce que la couchette (1) comporte sur au moins un côté une rainure ou un canal (3) pour recevoir l'agent de nettoyage qui s'écoule sur les côtés lors du repoussement ou du raclage, et en ce que la couchette (1) est équipée d'un récipient (9) pour recevoir l'agent de nettoyage repousée ou raclé.

8. Appareil de bronzage intégral selon l'une quelconque des Revendications précédentes,
**caractérisé** en ce qu'un affichage visuel relié au dispositif de nettoyage (2) est prévu, qui indique visuellement l'opération de nettoyage.

9. Appareil de bronzage intégral selon l'une quelconque des Revendications précédentes,
**caractérisé** en ce qu'une liaison est prévue entre le dispositif de nettoyage (2) et l'appareil de bronzage intégral, de telle facon que le dispositif de nettoyage (2) puisse être activé par la mise à l'arrêt de l'appareil de bronzage intégral.

10. Appareil de bronzage intégral selon l'une quelconque des Revendications précédentes,
**caractérisé** en ce que le dispositif de nettoyage (2) est orienté perpendiculairement à l'axe longitudinal de la couchette (1).

11. Appareil de bronzage intégral selon l'une quelconque des Revendications précédentes,
**caractérisé** en ce que l'ouverture de sortie (6) ou une buse prévue en plus est conformée en buse à vapeur.

12. Appareil de bronzage intégral selon l'une quelconque des Revendications précédentes,
**caractérisé** en ce que le dispositif de nettoyage (2) comporte au moins une réglette et/ou un cylindre de nettoyage (15), qui est au moins temporairement en contact avec la couchette (1) et qui comporte des brosses et/ou un matériau textile, de facon qu'un lustrage et/ou un traitement mécanique ultérieur de la couchette (1) soit possible après un traitement à la vapeur.

13. Appareil de bronzage intégral selon l'une quelconque des Revendications précédentes,
**caractérisé** en ce qu'un coussin de tête (11) est placé sous une membrane flexible (12) et en ce que le coussin de tête (11) est intégré de manière affleurante à la couchette (1) pendant une opération de nettoyage.

14. Appareil de bronzage intégral selon la Revendication 13,
**caractérisé** en ce que le coussin de tête (11) est organisé sous la forme d'une surface molle dans le plan de la couchette (1).

15. Appareil de bronzage intégral selon la Revendication 13 ou 14,
**caractérisé** en ce que sous le coussin de tête (11) est placé un étançon (13) et/ou un dispositif de surpression (14), par l'intermédiaire duquel la membrane (12) du coussin de tête (11) peut être ammenée dans une forme surélevée.
